# EUROPEAN PATENT APPLICATION

(11) **EP 4 458 379 A1**
(43) Date of publication of application: **06.11.2024**
(21) Application number: 22913168.5
(22) Date of filing: 09.06.2022
(51) Int. Cl.: A61L 15/18, A61L 15/26, A61L 15/28, A61L 15/42, A61L 15/44, A61L 15/46

(54) **ANTIBACTERIAL POLYURETHANE SPONGE AND PREPARATION METHOD THEREFOR**

(30) Priority: 31.12.2021 CN 202111669589
(71) Applicant: Roosin Medical Co., Ltd., Gaogang Taizhou, Jiangsu 225321 (CN)
(72) Inventor: CHEN, Dayong, Taizhou, Jiangsu 225321 (CN); CHU, Jiamian, Taizhou, Jiangsu 225321 (CN); CHU, Xingwu, Taizhou, Jiangsu 225321 (CN)
(74) Representative: Gill Jennings & Every LLP
(86) International application number: PCT/CN2022/097924
(87) International publication number: WO 2023/123879

(57) **Abstract**

The present invention relates to the technical field of medical dressings, in particular to an antibacterial polyurethane sponge and a preparation method therefor. An antibacterial dressing, which is obtained by means of adding an antibacterial effective component to a molded polyurethane sponge dressing in a soaking or spraying mode, has a poor antibacterial durability. According to the present invention, on the basis of the problem, a water dispersion system of an antibacterial agent is pre-dispersed in a polyurethane prepolymer and then foamed, which enables the antibacterial agent to be encapsulated in the porous structure of a polyurethane sponge and achieves the effect of slowly releasing antibacterial metal ions, so that the prepared antibacterial polyurethane sponge has a good antibacterial durability.

## Description

### Technical Field

The present invention relates to the technical field of medical dressings, in particular to an antibacterial polyurethane sponge and a preparation method therefor.

### Background Art

With the development and improvement of medical technology, there are more and more functional dressings related to wound care. Compared with traditional dressings, various antibacterial dressings for wound infection prevention or wound infection control have been more and more applied in clinics, and the effect of use has also been recognized by the majority of doctors and patients.

Antibacterial dressings on the market include foams, sponges, hydrogels, and other dressings. Antibacterial components in dressings are natural animal and plant extracts, soluble metal ion solutions, metal complexes, nanoscale metal powder inclusion materials, etc. Among them, foam or sponge dressings are the focus of current research. For example, Chinese invention patent No. CN104072798B discloses a preparation method of a nanoparticle-modified sponge dressing with an antibacterial function, comprising soaking a sponge as a base material in a soluble metal salt solution or/and a dispersion liquid of metal oxide nanoparticle and ultrasonically treating for combination to obtain the sponge dressing with an antibacterial function. For another example, Chinese invention patent No. CN201510995203.3 discloses a foam-based dressing and a preparation method therefor, wherein the foam-based dressing is composed of a base frame made of a sheet foam dressing and a gel material filled in cells of the base frame, and the foam-based dressing also contains copper ions, silver ions, or nano-silver with antibacterial properties.

Currently, sponge or foam dressings mainly use a soaking or spraying mode to add an antibacterial effective component, which has some disadvantages as follows:
(1) A soluble metal salt solution is added to the surface of a foam or sponge dressing in a soaking or spraying mode and then dried. When the dressing is applied on a wound, the soluble metal salts in the dressing rapidly release metal ions into the wound, which makes it difficult to control the release rate and concentration of the metal ions, has no long-lasting antibacterial effect, and is also easy to cause a large amount of metal ions to accumulate at the wound, resulting in excessive local concentration of ions, thereby causing skin discoloration.
(2) A nano-metal oxide antibacterial material is added to the surface of the dressing in the form of a gel, coating, or microsphere, which has a good antibacterial effect. However, metal nanoparticles have poor adhesion on the surface of the dressing, easily enter human blood, and accumulate in human tissues or organs, which are difficult to be metabolized and excreted and easily cause great toxicity.

### Summary of the Invention

With regard to the problems existing in the prior art, the technical problem to be solved by the present invention is that an antibacterial dressing, which is obtained by means of adding an antibacterial effective component to a molded sponge dressing in a soaking or spraying mode, has a poor antibacterial durability.

The technical solution used in the present invention to solve the technical problem is that the present invention provides an antibacterial polyurethane sponge comprising an ingredient A and an ingredient B, the mass ratio of the ingredient A to the ingredient B being 1:(0.5-5);
the ingredient A comprising the following components in parts by weight:

| | |
|---|---|
| antibacterial agent | 0.01-10 parts |
| suspending agent | 0.2-5 parts |
| wetting agent | 0.2-5 parts |
| stabilizer | 0.2-5 parts |
| water | 65-99 parts; |

the ingredient B being a polyurethane prepolymer;
the antibacterial polyurethane sponge being prepared by means of adding the ingredient A to the ingredient B prior to polyurethane foaming.

Specifically, the antibacterial agent is a metal oxide antibacterial agent.

Specifically, the metal oxide antibacterial agent is one or more of copper oxide, cuprous oxide, zinc oxide, and silver oxide.

Specifically, the suspending agent is a natural polymer suspending agent or a synthetic polymer suspending agent.

Specifically, the natural polymer suspending agent is gum arabic, tragacanth, apricot gum, peach gum, gum bletilla, agar, or chitosan.

Specifically, the synthetic polymer suspending agent is sodium carboxymethyl cellulose, methyl cellulose, carboxypropylmethyl cellulose, hydroxypropyl cellulose, hydroxyethyl cellulose, pyrrolidone, sodium acrylate, acrylate, or alpha-glucan.

Specifically, the wetting agent is one or more of glycerol, polyethylene glycol-2000, polyethylene glycol-3000, polyethylene glycol-3350, polyethylene glycol 4000, polyethylene glycol 6000, and polyethylene glycol 8000.

Specifically, the stabilizer is one or more of polysorbate 40, polysorbate 60, polysorbate 65, polysorbate 80, polysorbate 85, Span 20, Span 40, Span 60, Span 65, Span 80, Span 85, poloxamer PE6200, poloxamer PE6400, poloxamer PE6800, poloxamer PE9200, and poloxamer F68.

Specifically, the water is purified water, injection water, or deionized water.

Specifically, the polyurethane prepolymer has a viscosity of 6000-21000 mPa·s.

Specifically, the polyurethane prepolymer is Baymedix^{®}FP504, VORANATE^{™} T-80, or WanCURE TP611.

In another aspect, the present invention further provides a preparation method of the antibacterial polyurethane sponge as described above, comprising the following steps:
(1) mixing the antibacterial agent, the suspending agent, the wetting agent, the stabilizer, and the water uniformly according to the formulated amounts to obtain the ingredient A;
(2) simultaneously adding the ingredient A and the ingredient B in a mass ratio of 1 :(0.5-5) to a high-shear mixing device from different pipelines, stirring and mixing at a speed of 6000 rpm for 1-5 s to obtain an ingredient C; and
(3) rapidly extruding the ingredient C onto a release paper surface or into a mold for foaming, maturing, and then drying at 40-60°C for 20-50 min to obtain the antibacterial polyurethane sponge.

The present invention has the following beneficial effects.
(1) The antibacterial polyurethane sponge is prepared by adding the ingredient A (a water dispersion system of an antibacterial agent) to the ingredient B (a prepolymer) prior to polyurethane foaming, namely adding a water dispersion system of the antibacterial agent to the prepolymer prior to polyurethane foaming, which enables the antibacterial agent to be encapsulated in the porous structure of the polyurethane sponge and achieves the effect of slowly releasing antibacterial metal particles, so that the prepared antibacterial polyurethane sponge has a good antibacterial durability.
(2) According to the present invention, a certain amount of suspending agent is added to a water dispersion system of the antibacterial metal oxide (metal oxide antibacterial agent), so that the antibacterial agent can be better dispersed in the polyurethane sponge, which is advantageous to further improve the antibacterial property of the polyurethane sponge dressing.
(3) The water dispersion system of the antibacterial agent of the present invention is easy to be cleaned and can be thoroughly cleaned using common solvents such as water, ethanol, etc., which will not contaminate the inner walls of a reactor, and can be replaced with different types of metal oxide antibacterial agents at any time.

### Detailed Description of the Invention

The present invention will now be described in further detail with reference to Examples.

### Example 1

0.01 g of copper oxide with a particle size of 130 nm, 0.2 g of gum arabic, 0.2 g of poloxamer PE6800, 0.2 g of PEG-2000, and 65 g of injection water were mixed uniformly to obtain an ingredient A;
(2) 100 g of ingredient A and 50 g of WanCURE TP611 were added simultaneously to a high-shear mixing device from different pipelines, stirred and mixed at a speed of 6000 rpm for 2 s to obtain an ingredient C; and
(3) the ingredient C was rapidly extruded onto a release paper surface to form a foam, and compression rollers were used to form a gap to adjust the thickness of the foam to 10 mm; the foam was matured at room temperature for 4 min, and then dried at 40°C for 20 min to obtain an antibacterial polyurethane sponge with a foaming density of 150 kg/m³ and an open cell diameter of 0.01 mm.

### Example 2

(1) 0.5 g of cuprous oxide with a particle size of 110 nm, 1.0 g of sodium carboxymethyl cellulose, 0.5 g of polysorbate 60, 1 g of PEG-3000, and 97 g of purified water were mixed uniformly to obtain an ingredient A;
(2) 100 g of ingredient A and 100 g of WanCURE TP611 were added simultaneously to a high-shear mixing device from different pipelines, stirred and mixed at a speed of 6000 rpm for 5 s to obtain an ingredient C; and
(3) the ingredient C was rapidly extruded onto a release paper surface to form a foam, and the thickness of the foam was adjusted to 10 mm; the foam was matured at room temperature for 5 min, and then dried at 50°C for 30 min to obtain an antibacterial polyurethane sponge with a foaming density of 130 kg/m³ and an open cell diameter of 0.03 mm.

### Example 3

(1) 1.0 g of silver oxide with a particle size of 150 nm, 1.0 g of sodium carboxymethyl cellulose, 1.0 g of polysorbate 60, 2.0 g of PEG-3000, and 95.0 g of purified water were mixed uniformly to obtain an ingredient A;
(2) 100 g of ingredient A and 400 g of Baymedix^{®}FP504 were added simultaneously to a high-shear mixing device from different pipelines, stirred and mixed at a speed of 6000 rpm for 5 s to obtain an ingredient C; and
(3) the ingredient C was rapidly extruded into a mold to form a block foam; the block foam was matured at room temperature for 7 min, and then dried at 40°C for 50 min to obtain a block antibacterial polyurethane sponge with a foaming density of 90 kg/m³ and an open cell diameter of 0.05 mm.

### Example 4

(1) 1.0 g of zinc oxide with a particle size of 160 nm, 1.0 g of tragacanth, 0.5 g of polysorbate 20, 3 g of sorbitan oleate (Span 80), and 94.5 g of purified water were mixed uniformly to obtain an ingredient A;
(2) 100 g of ingredient A and 90 g of VORANATE^{™} T-80 were added simultaneously to a high-shear mixing device from different pipelines, stirred and mixed at a speed of 6000 rpm for 5 s to obtain an ingredient C; and
(3) the ingredient C was rapidly extruded into a mold to form a block foam; the block foam was matured at room temperature for 5 min, and then dried at 60°C for 20 min to obtain a block antibacterial polyurethane sponge with a foaming density of 140 kg/m³ and an open cell diameter of 0.03 mm.

### Example 5

(1) 10 g of silver oxide with a particle size of 150 nm, 5 g of agar, 5 g of polysorbate 85, 5 g of PEG-8000, and 85 g of deionized water were mixed uniformly to obtain an ingredient A;
(2) 100 g of ingredient A and 500 g of Baymedix^{®}FP504 were added simultaneously to a high-shear mixing device from different pipelines, stirred and mixed at a speed of 6000 rpm for 5 s to obtain an ingredient C; and
(3) the ingredient C was rapidly extruded into a mold to form a block foam; the block foam was matured at room temperature for 8 min, and then dried at 40°C for 50 min to obtain a block antibacterial polyurethane sponge with a foaming density of 85 kg/m³ and an open cell diameter of 0.05 mm.

### Example 6

(1) 5 g of cuprous oxide with a particle size of 120 nm, 3 g of alpha-glucan, 2 g of polysorbate 40, 2 g of PEG-6000, and 88 g of purified water were mixed uniformly to obtain an ingredient A;
(2) 100 g of ingredient A and 200 g of WanCURE TP611 were added simultaneously into a high-shear mixing device from different pipelines, stirred and mixed at a speed of 6000 rpm for 5 s to obtain an ingredient C; and
(3) the ingredient C was rapidly extruded onto a release paper surface to form a foam, and pressing plates were used to form a gap to adjust the thickness of the foam to 10 mm; the foam was matured at room temperature for 8 min, and then dried at 50°C for 30 min to obtain an antibacterial polyurethane sponge with a foaming density of 90 kg/m³ and an open cell diameter of 0.05 mm.

Comparative Example 1 was the same as Example 3, except that no suspending agent was added in Comparative Example 1.

Example 7 was the same as Example 3, except that the suspending agent in Example 7 was gum arabic.

Example 8 was the same as Example 3, except that the suspending agent in Example 8 was tragacanth.

Example 9 was the same as Example 3, except that the suspending agent in Example 9 was agar. Example 10 was the same as Example 3, except that the suspending agent in Example 10 was alpha-glucan.

Comparative Example 2 was the same as Example 3, except that the surfactant polyoxyethylene-polyoxypropylene copolymer F68 was used instead of tragacanth in Comparative Example 2.

Comparative Example 3 was the same as Example 3, except that in Comparative Example 3, no suspending agent was added, the stabilizer was silicone surfactant L64, and the wetting agent was the surfactant polyoxyethylene-polyoxypropylene copolymer F127.

### Comparative Example 4

Unlike Example 3, in Comparative Example 4, a dried polyurethane sponge was first prepared, and a water dispersion system of an antibacterial agent was then added, specifically:
Step 1: 1.0 g of sodium carboxymethyl cellulose was dispersed in 98.0 g of purified water for soaking, and after complete dissolution, 1.0 g of silver oxide with a particle size of 150 nm was added thereto and mixed uniformly to form a suspension;
Step 2: preparation of the polyurethane sponge:
   (1) 2.0 g of PEG-3000, 1.0 g of polysorbate 60, and 95.0 g of purified water were mixed uniformly to obtain an ingredient A-1;
   (2) 98 g of ingredient A-1 and 400 g of Baymedix^{®}FP504 were added simultaneously to a high-shear mixing device from different pipelines, stirred and mixed at a speed of 6000 rpm for 5 s to obtain an ingredient C-1; and
   (3) the ingredient C-1 was rapidly extruded into a mold to form a block foam; the block foam was matured at room temperature for 7 min, and then dried at 40°C for 50 min to obtain a block antibacterial polyurethane sponge with a foaming density of 90 kg/m³ and an open cell diameter of 0.05 mm;
Step 3: the suspension obtained in step (1) was sprayed onto the polyurethane sponge obtained in step (2) at a uniform speed and dried to form an antibacterial polyurethane sponge.

### Comparative Example 5

Unlike Example 3, in Comparative Example 5, the proportion of the sodium carboxymethyl cellulose added was less than 0.2 g, specifically:
(1) 1.0 g of silver oxide with a particle size of 150 nm, 0.1 g of sodium carboxymethyl cellulose, 1.0 g of polysorbate 60, 2.0 g of PEG-3000, and 95.9 g of purified water were mixed uniformly to obtain 100 g of ingredient A;
(2) 100 g of ingredient A and ingredient B, 400 g of Baymedix^{®}FP504, were added simultaneously to a high-shear mixing device from different pipelines, stirred and mixed at a speed of 6000 rpm for 5 s to obtain an ingredient C; and
(3) the ingredient C was rapidly extruded into a mold to form a block foam; the block foam was matured at room temperature for 7 min, and then dried at 40°C for 50 min to obtain a block antibacterial polyurethane sponge with a foaming density of 90 kg/m³ and an open cell diameter of 0.05 mm.

### Antibacterial test:

The antibacterial polyurethane sponges obtained in Examples 1-6 and Comparative Examples 1-9 were tested for antibacterial properties. The specific test results are shown in Table 1.

The antibacterial rate was tested according to the test standard - US AATCC 100-2019 evaluation method of antibacterial textiles.

**Table 1: Antibacterial test results of each of the Examples and Comparative Examples**

| Test item | Escherichia coli | | Staphylococcus aureus | | Candida albicans | |
|---|---|---|---|---|---|---|
| | Antibacterial rate (%) | Antibacterial durability | Antibacterial rate (%) | Antibacterial durability | Antibacterial rate (%) | Antibacterial durability |
| Example 1 | 99.98 | 168h | 99.97 | 168h | 99.90 | 168h |
| Example 2 | 99.99 | 168h | 99.99 | 168h | 99.99 | 168h |
| Example 3 | 99.99 | 168h | 99.99 | 168h | 99.99 | 168h |
| Example 4 | 99.99 | 168h | 99.99 | 168h | 99.98 | 168h |
| Example 5 | 99.99 | 168h | 99.99 | 168h | 99.99 | 168h |
| Example 6 | 99.99 | 168h | 99.99 | 168h | 99.99 | 168h |
| Comparative Example 1 | 90.19 | 168h | 92.99 | 168h | 86.83 | 168h |
| Example 7 | 99.95 | 168h | 99.97 | 168h | 99.95 | 168h |
| Example 8 | 99.94 | 168h | 99.99 | 168h | 99.87 | 168h |
| Example 9 | 99.97 | 168h | 99.96 | 168h | 99.94 | 168h |
| Example 10 | 99.98 | 168h | 99.98 | 168h | 99.91 | 168h |
| Comparative Example 2 | 93.29 | 168h | 91.97 | 168h | 90.96 | 168h |
| Comparative Example 3 | 94.98 | 168h | 96.97 | 168h | 93.90 | 168h |
| Comparative Example 4 | 83.26 | 168h | 81.91 | 168h | 80.69 | 168h |
| Comparative Example 5 | 97.89 | 168h | 97.97 | 168h | 97.90 | 168h |

### Determination of metal ion contents:

A sample was firstly subjected to digestion and acidolysis treatment, and then determined according to the method specified in GB/T23942-2009, "Chemical Reagent - General rules for inductively coupled plasma atomic emission spectrometry". The test data are shown in detail in Table 2.

**Table 2: Determination of metal ion release concentration in each of the Examples and Comparative Examples**

| Test item | Metal ion release concentration (in ppm) | | | | | | | |
|---|---|---|---|---|---|---|---|---|
| | 0h | 24h | 48h | 72h | 96h | 120h | 144h | 168h |
| Example 1 | 14 | 26 | 23 | 24 | 29 | 31 | 23 | 30 |
| Example 2 | 18 | 42 | 49 | 45 | 49 | 42 | 43 | 48 |
| Example 3 | 15 | 46 | 40 | 48 | 49 | 43 | 46 | 44 |
| Example 4 | 13 | 34 | 33 | 35 | 38 | 36 | 38 | 39 |
| Example 5 | 32 | 146 | 140 | 149 | 152 | 156 | 155 | 152 |
| Example 6 | 29 | 79 | 78 | 73 | 77 | 70 | 71 | 74 |
| Comparative Example 1 | 11 | 24 | 23 | 20 | 22 | 20 | 19 | 16 |
| Example 7 | 13 | 38 | 36 | 38 | 44 | 42 | 41 | 40 |
| Example 8 | 15 | 36 | 41 | 38 | 37 | 43 | 39 | 41 |
| Example 9 | 14 | 40 | 42 | 44 | 40 | 41 | 43 | 40 |
| Example 10 | 15 | 36 | 40 | 38 | 39 | 33 | 36 | 36 |
| Comparative Example 2 | 15 | 26 | 24 | 25 | 22 | 23 | 24 | 22 |
| Comparative Example 3 | 15 | 26 | 28 | 23 | 26 | 24 | 25 | 24 |
| Comparative Example 4 | 225 | 510 | 287 | 152 | 86 | 34 | 12 | 9 |
| Comparative Example 5 | 12 | 27 | 29 | 31 | 30 | 28 | 26 | 29 |

It can be seen from the above results that an antibacterial polyurethane sponge provided in the present invention has a good antibacterial effect and antibacterial durability; also, the antibacterial polyurethane sponge in each of the Examples can release stably and uniformly metal ions, so that the release rate of metal ions can be controlled and slow-released, avoiding excessive local concentration of ions due to the rapid release of a large number of metal ions into a wound. However, compared with Example 3, the antibacterial polyurethane sponge provided in Comparative Example 1, in which no suspending agent was added during the preparation process, has a poor antibacterial durability, a reduced release concentration of metal ions in the same time, and a poor antibacterial effect. The antibacterial polyurethane sponges provided in Examples 7-10, in which the suspending agents were changed to gum arabic, tragacanth, agar, and alpha-glucan, respectively, still have strong antibacterial properties, and the antibacterial rate at 168 h is close to Example 3, falling within the scope of the present invention. The antibacterial polyurethane sponge formed by the solution provided in Comparative Example 2, in which the surfactant polyoxyethylene-polyoxypropylene copolymer F68 was used instead of tragacanth, although the metals are stably released, has a reduced antibacterial rate and a reduced release concentration of metal ions due to the polyoxyethylene-polyoxypropylene copolymer F68 added has no suspension effect, which affects the antibacterial effect and the antibacterial durability. The solution provided in Comparative Example 3 is that no suspending agent was added, the stabilizer was silicone surfactant L64, and the wetting agent was surfactant polyoxypropylene-polyoxyethylene copolymer F127, which causes both the antibacterial effect and the release concentration of metal ions to be reduced compared with those in Example 3. The solution provided in Comparative Example 4 is that a polyurethane sponge was first prepared, and a water dispersion system containing antibacterial metal ions was then added, which shows that the metal ions were rapidly released, the release rate fluctuated too much, and the release of metal ions had no durability, thereby affecting the antibacterial rate and durability, the durability and antibacterial rate being significantly reduced compared with those in Example 3. The solution provided in Comparative Example 5 is that the amount of the suspending agent sodium carboxymethyl cellulose added was less than the range of 0.2-5 parts, which results in a significant reduction of antibacterial rate after 168 h compared with Example 3, although the metal irons are stably released.

Taking the ideal embodiment of the present invention as inspiration, various changes and modifications may be made by those skilled in the art from the above description without departing from the scope of the present invention. The technical scope of the present invention is not limited to the description, but must be determined according to the scope of the claims.

## Claims

1. An antibacterial polyurethane sponge, comprising an ingredient A and an ingredient B, the mass ratio of the ingredient A to the ingredient B being 1:(0.5-5);
the ingredient A comprising the following components in parts by weight:
| | |
|---|---|
| antibacterial agent | 0.01-10 parts |
| suspending agent | 0.2-5 parts |
| wetting agent | 0.2-5 parts |
| stabilizer | 0.2-5 parts |
| water | 65-99 parts; |
the ingredient B being a polyurethane prepolymer;
the antibacterial polyurethane sponge being prepared by means of adding the ingredient A to the ingredient B prior to polyurethane foaming.

2. The antibacterial polyurethane sponge according to claim 1, wherein the antibacterial agent is a metal oxide antibacterial agent.

3. The antibacterial polyurethane sponge according to claim 2, wherein the metal oxide antibacterial agent is one or more of copper oxide, cuprous oxide, zinc oxide, and silver oxide.

4. The antibacterial polyurethane sponge according to claim 1, wherein the suspending agent is a natural polymer suspending agent or a synthetic polymer suspending agent.

5. The antibacterial polyurethane sponge according to claim 4, wherein the natural polymer suspending agent is gum arabic, tragacanth, apricot gum, peach gum, gum bletilla, agar, or chitosan; the synthetic polymer suspending agent is sodium carboxymethyl cellulose, methyl cellulose, carboxypropylmethyl cellulose, hydroxypropyl cellulose, hydroxyethyl cellulose, pyrrolidone, sodium acrylate, acrylate, or alpha-glucan.

6. The antibacterial polyurethane sponge according to claim 1, wherein the wetting agent is one or more of polyethylene glycol-2000, polyethylene glycol-3000, polyethylene glycol-3350, polyethylene glycol 4000, polyethylene glycol 6000, and polyethylene glycol 8000.

7. The antibacterial polyurethane sponge according to claim 1, wherein the stabilizer is one or more of polysorbate 40, polysorbate 60, polysorbate 65, polysorbate 80, polysorbate 85, poloxamer PE6200, poloxamer PE6400, poloxamer PE6800, poloxamer PE9200, and poloxamer F68.

8. The antibacterial polyurethane sponge according to claim 1, wherein the polyurethane prepolymer has a viscosity of 6000-21000 mPa·s.

9. The antibacterial polyurethane sponge according to claim 8, wherein the polyurethane prepolymer is Baymedix^{®}FP504, VORANATE^{™} T-80, or WanCURE TP611.

10. A preparation method of the antibacterial polyurethane sponge according to any one of claims 1-9, comprising the following steps:
(1) mixing the antibacterial agent, the suspending agent, the wetting agent, the stabilizer, and the water uniformly according to the formulated amounts to obtain the ingredient A;
(2) simultaneously adding the ingredient A and the ingredient B in a mass ratio of 1:(0.5-5) to a high-shear mixing device from different pipelines, stirring and mixing at a speed of 6000 rpm for 1-5 s to obtain an ingredient C; and
(3) rapidly extruding the ingredient C onto a release paper surface, a release film surface, or into a mold for foaming, maturing, and then drying at 40-60°C for 20-50 min to obtain the antibacterial polyurethane sponge.
